(19) Europäisches Patentamt
European Patent Office
Office européen des brevets

(11) Veröffentlichungsnummer: **0 137 361**
**B1**

(12) **EUROPÄISCHE PATENTSCHRIFT**

(45) Veröffentlichungstag der Patentschrift: **16.05.90**

(51) Int. Cl.$^5$: **C 07 K 7/40**, A 61 K 37/26

(21) Anmeldenummer: **84111058.8**

(22) Anmeldetag: **17.09.84**

(54) **In Position B 30 modifizierte Insulin-Derivate, Verfahren zu deren Herstellung und deren Verwendung sowie pharmazeutische Mittel zur Behandlung des Diabetes mellitus.**

(30) Priorität: **23.09.83 DE 3334407**

(43) Veröffentlichungstag der Anmeldung:
**17.04.85 Patentblatt 85/16**

(45) Bekanntmachung des Hinweises auf die Patenterteilung:
**16.05.90 Patentblatt 90/20**

(84) Benannte Vertragsstaaten:
**AT BE CH DE FR GB IT LI LU NL SE**

(56) Entgegenhaltungen:
**EP-A-0 056 951**
**EP-A-0 140 084**
**FR-A-2 313 939**
**GB-A-2 069 502**
**JP-B-5 767 548**

**Diabetes 1981 , 30(6) , 519-22**
**Dept Chem 1980 S 157-162**
**CHEMICAL ABSTRACTS , Band 97,Nr.23,6.Dezember 1982,Seite 616,Ref.Nr.198580h;Columbus,Ohio,US & JP-A-82 67 548**

(73) Patentinhaber: **HOECHST AKTIENGESELLSCHAFT**
**Postfach 80 03 20**
**D-6230 Frankfurt am Main 80 (DE)**

(72) Erfinder: **Grau, Ulrich, Dr.**
**Zeil 17**
**D-6238 Hofheim am Taunus (DE)**
Erfinder: **Geiger, Rolf, Prof. Dr.**
**Heinrich-Bleicher-Strasse 33**
**D-6000 Frankfurt am Main 50 (DE)**
Erfinder: **Obermeier, Rainer, Dr.**
**Langenhainer Weg 14**
**D-6234 Hattersheim am Main (DE)**

(56) Entgegenhaltungen:
**CHEMICAL ABSTRACTS , Band 97,Nr.17,25.Oktober 1982,Seite 95,Ref.Nr. 138818g; Columbus , Ohio ,US M.KOBAYASHI et al.: "Immunoreactivity and biologic activity of semi-synthetic (Leu B-30)-insulin.Potential value in the treatment of insulin antibody-mediated insulin resistance"**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 137 361 B1

(56) Entgegenhaltungen:

CHEMICAL ABSTRACTS, Band 98, Nr. 19, 9. Mai 1983, Seite 552, Ref.Nr. 161148t; Columbus, Ohio, US H.G.GATTNER et al.: "Trypsin catalized peptide synthesis: Modification of the B-chain C-terminal region of insulin"

CHEMICAL ABSTRACTS, Band 95, Nr. 11, 14. September 1981, Seite 709, Ref.Nr. 98302j; Columbus, Ohio, US T.OKA et al.: "Enzymic preparation of B-30 substituted porcine insulins and their antigenic specificities"

## Beschreibung

Bei der Therapie des Diabetes mellitus werden heute im allgemeinen Zubereitungen des blutzuckersenkenden Hormons Insulin parenteral verabreicht. Die spezielle Natur des Insulins und dessen Metabolismus bringen es mit sich, daß die Wirkungsdauer einer einfachen Lösung nur sehr kurz ist, daß also zur anhaltenden Blutzuckerkontrolle beim Diabetiker entweder eine Dauer-Infusion mit Dosiergeräten, mehrfache tägliche Injektionen oder eine verzögert wirksame Insulin-zubereitung appliziert werden müssen. Als Verzögerungsprinzipien sind dabei von besonderer Bedeutung solche Zustandsformen des Insulins, die am Ort der Injektion schwerlöslich sind (z.B. kristallin oder amorph). Dazu zu rechnen sind beispielsweise Zink-Insulinkristalle oder Protamin-Insulin-Kristalle, die während ihrer langsamen Wiederauflösung über einen gewissen Zeitraum Insulin freisetzen.

Nun hat es sich bei der Therapie als äußerst hilfreich erwiesen, verschiedene Insulinpräparate zur Verfügung zu haben, die in ihrer Wirkungscharakteristik den Bedürfnissen des einzelnen Patienten möglichst nahe kommen. Im Zusammenhang mit nicht-optimaler Einstellung werden neben unmittelbaren Effekten wie Hyper- oder Hypoglykämien insbesondere die diabetischen Spätkomplikationen diskutiert, zu dennen Retinopathie, Neuropathie, Nephropatie, Mikro- und Makroangiopathie zählen.

Der Insulinmangel beim Diabetiker führt dazu, daß der Körper sein natürliches hormonelles Gleichgewicht nicht mehr erreichen kann.

Aufgabe der Erfindung ist die Bereitstellung eines Insulin-Derivates bzw. eines entsprechenden pharmazeutischen Mittels, durch das man sich dem natürlichen hormonellen Gleichgewicht in einem diabetischen Zustand besser annähern kann und wodurch dieses sich besser aufrechterhalten läßt als durch die Verabreichung von Insulin in den bisher üblichen Formen.

Diese Aufgabe wird nun erfindungsgemäß gelöst durch die Bereitstellung eines Arzneimittels, welches mindestens ein Insulin-Derivat mit einer neutralen, genetisch kodierbaren Aminosäure in B30-Position der B-Kette enthält, wobei die C-terminale Carboxylgruppe der B30-Aminosäure nicht in freier Form vorliegt, sondern mit einer physiologisch verträglichen, neutralen Gruppe — vorzugsweise einer Ester- oder Amidgruppe — blockiert ist.

einige in Position B30 veresterte Humaninsuline sowie Verfahren zu ihrer Herstellung wurden schon als Zwischenprodukte bei der Semisynthese von Humaninsulin beschreiben. So ist Humaninsulin-$Thr^{B30}$-OBut beispielsweise aus den US—A—4 320 196 und US—A—4 320 197 bekannt. In der GB—A—2 069 502 werden Humaninsulin-$Thr^{B30}$-OMe, Humaninsulin-$Thr^{B30}$-OEt, Humaninsulin-$Thr^{B30}$-O-(2,4,6-Trimethylbenzyl) sowie Humaninsulin-$Thr^{B30}(Bu^t)$-$OBu^t$ beschreiben. Aus der EP—A—45 187 ist Humaninsulin-(B30)-amid bekannt. Die Eignung dieser Insulin-Derivate — ohne Deblockierung — zur Behandlung von Diabetes mellitus ist in den genannten Druckschriften jedoch nicht beschreiben.

Bei biologischen Tests im Zusammenhang mit Untersuchungen über die Krankheit Diabetes mellitus wurden aber auch bereits Insulin-Derivate mit blockierter Carboxylgruppe an der B30-Aminosäure geprüft; ess handelt sich speziell um Des-B30-Humaninsulin-$Leu^{B30}$-$NH_2$, Des-B30-Humaninsulin-$^{B30}$-$NH_2$ und Des-B30-Humaninsulin-$Phe^{B30}$-$NH_2$; vgl. M. Kobayashi et al., Diabetes 1981, 30(6), 519—22, ref. in C.A. Vol. 97 Nr. 17 (1982), S, 95, Ref. Nr. 138818g, JP—B—57—67548, ref. in C.A. Vol. 97 Nr. 23 (1982), S. 616, Ref.-Nr. 198 580h, und T. Oka et al., Pept. Chem. 1980, S. 157—162, ref. in C.A. Vol. 95, Nr. 11 (1981), S. 709, Ref.-Nr. 98302j.

Über das besondere Wirkungsprofil der Insulin-Derivate mit blockierter Carboxylgruppe an der B30-Aminosäure ist in diesen Literaturstellen nichts ausgesagt.

Die Erfindung betrifft nun ein Arzneimittel mit verzögerter Wirkung zur Behandlung von Diabetes mellitus, bestehend aus mindestens einem Insulin-Derivat der Formel I

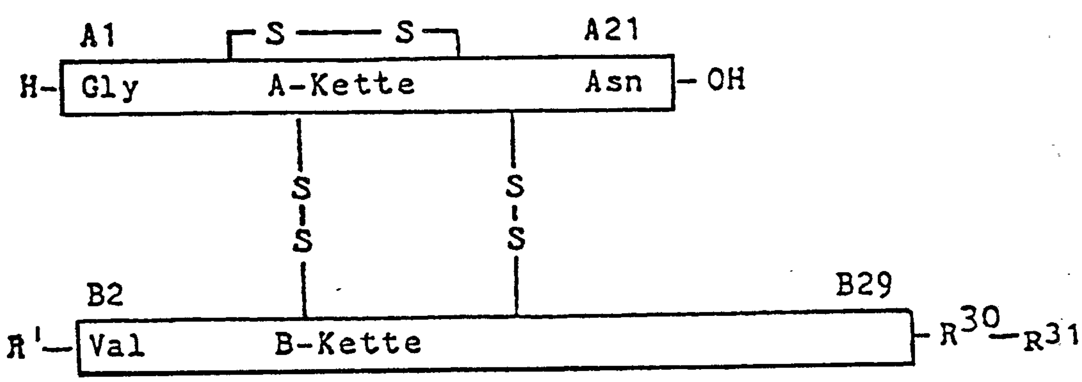

(I)

in welcher

$R^1$ H oder H-Phe bedeutet,

$R^{30}$ für den Rest einer neutralen, genetisch kodierbaren L-Aminosäure, deren gegebenenfalls vorhandene OH-Gruppe frei oder durch eine physiologisch verträgliche Gruppe geschützt vorliegen kann, steht und

$R^{31}$ eine physiologisch verträgliche, die Carboxygruppe blockierende neutrale Gruppe bedeutet, wobei jedoch Des-B30-Humaninsulin-$Leu^{B30}$-$NH_2$, Des-B30-Humaninsulin-$Gly^{B30}$-$NH_2$ und Des-B30-Humaninsulin-$Phe^{B30}$-$NH_2$ ausgenommen sind, und einem pharmazeutisch unbedenklichen Träger.

Genetisch kodierbar sind die folgenden L-Aminosäuren: *Gly, Ala, Ser, Thr, Val, Leu, Ile,* Asp, *Asn,* Glu, *Gln, Cys, Met,* Arg, Lys, His, *Tyr, Phe,* Trp, *Pro* (neutrale Aminosaüren unterstrichen).

Unter neutralen Gruppen, die die freie Carboxyfunktion am C-terminalen Ende der B-kette in den Verbindungen der Formel I blockieren, versteht man physiologisch verträgliche ungeladene Gruppen, vor allem Ester- und Amidgruppen, oder andere COOH-Schutzgruppen, wie sie beispielsweise im Bodanszky et al., Peptide Synthesis, 2nd Edition (1976) John Wiley & Sons beschreiben werden, vorzugsweise Gruppen der Formel $-NR^aR^b$ oder $-OR^c$, worin $R^a$ und $R^b$ gleich oder verschieden sind und für Wasserstoff, $(C_1-C_6)$-Alkyl, $(C_3-C_8)$-Cycloalkyl, $(C_6-C_{10})$-Aryl, $(C_7-C_{11})$-Aralkyl, $(C_3-C_9)$-Heteroaryl oder $-(CH_2-CH_2-O)_mR$ mit $m = 1$ bis etwa 120 stehen und $R = (C_1-C_4)$-Alkyl, wobei diese Gruppen im Alkylteil auch durch einen oder mehrere gleiche oder verschiedene Substituenten aus der Reihe Halogen, Nitro, $(C_1-C_4)$-Alkoxy, Methylendioxy und $(C_1-C_4)$-Alkyl substituiert sein können oder $R^a$ und $R^b$ gemeinsam für $-[CH_2]_n-$ mit $n = 4-6$ stehen, worin eine Methylengruppe auch durch O oder NH ersetzt sein kann, und $R^c$ für $(C_1-C_6)$-Alkyl, $(C_3-C_8)$-Cycloalkyl, $(C_6-C_{10})$-Aryl, $(C_7-C_{11})$-Aralkyl, $(C_3-C_9)$-Heteroaryl oder den vorstehend definierten Rest $(CH_2CH_2-O-)_mR$ stehen, wobei die Arylreste wie bei $R^a$ und $R^b$ substituiert sein können.

Im obigen Zusammenhang und auch in der Folge wird unter $(C_1-C_6)$-Alkyl beispielsweise Methyl, Ethyl, Propyl, Isopropyl, Butyl, tert.-Butyl, Amyl oder Hexyl, unter $(C_3-C_8)$-Cycloalkyl beispielsweise Cyclopropyl, Cyclobutyl usw., unter $(C_6-C_{10})$-Aryl beispielsweise Phenyl oder Naphthyl, vorzugsweise Phenyl, unter $(C_7-C_{11})$-Aralkyl beispielsweise Benzyl, Phenethyl u.ä.,

unter $(C_3-C_9)$-Heteroaryl, beispielsweise Pyridyl, Pyrrolidinyl, Pyrimidiyl, Morpholinyl, Pyrazinyl, Imidazolyl, Indolyl oder Chinolinyl,

unter $(C_1-C_6)$-Alkanoyl beispielsweise Formyl, Acetyl, Propionyl, Butyryl usw., und

unter $(C_7-C_{11})$-Aroyl beispielsweise Benzoyl, Naphthoyl oder Toluyl verstanden.

Arzneimittel mit Insulin-Derivaten der Formel I, die in Position B1 Phenylalanin tragen, sind besonders bevorzugt. Weiterhin ist der Einsatz von Insulin-Derivaten bevorzugt, die in der Position B30 Ala, Thr oder Ser aufweisen.

Die A-Kette und die Kette (B2-30) der Insulin-Derivate der Formel I weisen zweckmäßigerweise die Sequenz des Rinder- oder Schweineinsulins, insbesondere aber die des Humaninsulins auf.

Bevorzugt ist in den Insulin-Derivaten der Formel I auch $R^{31} = -O(CH_2CH_2-O)_mR$ (m und R s. vorher) bzw. $= (C_1-C_6)$-Alkoxy oder $(C_7-C_{11})$-Aralkoxy.

Falls in den Aminosäuren in Position B30 noch eine Hydroxylgruppe vorhanden ist (was im Falle von Ser, Thr und Tyr zutrifft), kann diese frei oder geschützt sein durch physiologisch verträgliche Gruppen, bevorzugt aus der Reihe $(C_1-C_6)$-Alkyl, $(C_3-C_8)$-Cycloalkyl, $(C_6-C_{10})$-Aryl, $(C_8-C_{11})$-Aralkyl, $C_3-C_9$)-Heteroaryl, $(C_1-C_6)$-Alkanoyl und $(C_7-C_{11})$-Aroyl; auch andere, in der Peptidchemie gängige, physiologisch verträgliche Reste (vgl. z.B. Bodanszky et al., loc cit. . . .) können als Schutzgruppen dienen. Besonders bevorzugt sind jedoch $(C_1-C_6)$-Alkyl-, $(C_1-C_6)$-Alkanoyl- und $(C_7-C_{11})$-Aralkyl-Schutzgruppen.

In der Reihe der für die erfindungsgemäßen Arzneimittel in Frage kommenden Insulin-Derivate der Formel I seien beispielsweise die nachstehenden Verbindungen erwähnt, ohne die Erfindung auf diese einzuschränken:

Des-$Phe^{B1}$-Schweineinsulin-(B30)-$OBu^t$
Des-$Phe^{B1}$-Humaninsulin-(B30)-$OBu^t$
Des-$Phe^{B1}$-Schweineinsulin-(B30)-$OCH_3$
Des-$Phe^{B1}$-Humaninsulin-(B30)-OBz
Schweineinsulin-(B30)-O-Cyclohexyl

Humaninsulin-(B30)-O- 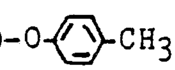

Rinderinsulin-(B30)-$OCH_3$
Schweineinsulin-(B30)-O-$(CH_2-CH_2-O-)_{20}C_2H_5$
Humaninsulin-(B30)-NH-$C_2H_5$
Des-$Thr^{B30}$-Humaninsulin-$Val^{B30}$-O-$C_2H_5$
Des-$Thr^{B30}$-Humaninsulin-$Val^{B30}$-$NH_2$

Humaninsulin-(B30) - 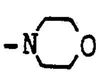

Humaninsulin-(B30)-OBz
Humaninsulin-(B30)-O-$CH_2$-$CH_2$

Humaninsulin-(B30)—NH—$(CH_2—CH_2—O)_{30}—CH_3$

Humaninsulin-(B30)—$N(CH_3)_2$ - 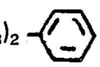

Humaninsulin-$Thr^{B30}(Et)OEt$
Humaninsulin-$Thr^{B30}(CH_3)OCH_3$
Humaninsulin-(B30)—O—$[CH_2]_5CH_3$
Humaninsulin-Thr(Ac)O—Et
Humaninsulin-Thr(Bz)$OBu^t$

Humaninsulin-(B30) 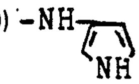

Humaninsulin-(B30) 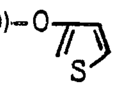

Humaninsulin-$Thr^{B30}$(FOR)—OPr
Humaninsulin-(B30)—$NH_2$

Die Insulin-Derivate der Formel I können dadurch hergestellt werden, daß man

a) ein Des-Octapeptid (B23-30)-Insulin der Formel II,

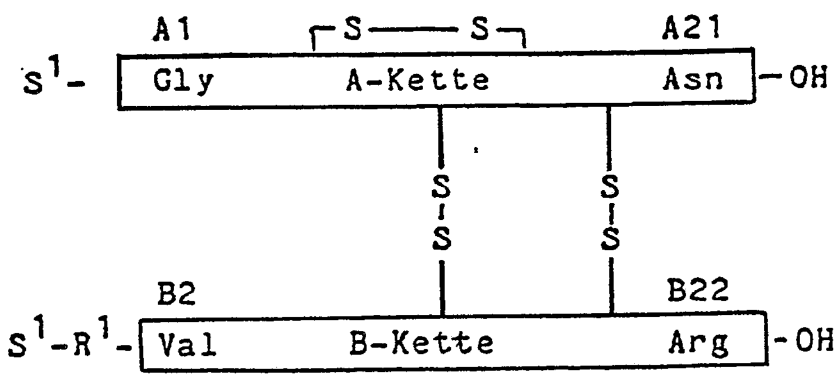

in der $R^1$ Phe oder eine Bindung und $S^1$ eine protonensolvolytisch oder durch β-Eliminierung abspaltbare Aminoschutzgruppe wie den tert.-Butyloxycarbonyl-(Boc), den tert.-Amyloxy- carbonyl-(Aoc) oder den Methylsulfonylethyloxycarbonyl-(Msc)-rest bedeuten, kondensiert mit einem Peptid der Formel III

$$H\text{-}Gly\text{-}Phe\text{-}Phe\text{-}Tyr(S^2)\text{-}Thr(S^2)\text{-}Pro\text{-}Lys(S^3)\text{-}R^{30}\text{-}R^{31} \qquad (III)$$

in welcher $R^{30}$ oder $R^{31}$ die oben definierten Bedeutungen haben, $S^2$ für Wasserstoff, Bzl oder $Bu^t$ und $S^3$ für eine Urethanschutzgruppe, wie Boc, Moc, Fmoc oder Z stehen, und vorhandene Schutzgruppen in an sich bekannter Weise abspaltet, oder

b) ein Des-B30-Insulin der Formel I, in welcher $R^1$ für H oder H-Phe und der C-Terminus $R^{30}$-$R^{31}$ zusammen für OH stehen, in Gegenwart von Trypsin oder einer trypsinähnlichen Endopeptidase umsetzt mit einer Verbindung der Formel IV

$$H—R^{30}—R^{31} \qquad (IV)$$

in der $R^{30}$ und $R^{31}$ die oben definierten Bedeutungen haben und anschließend gegebenenfalls vorhandene Schutzgruppen in an sich bekannter Weise abspaltet und die nach a) oder b) erhaltenen Verbindungen gegebenenfalls in ihre physiologisch verträglichen Salze überführt.

Bei der Verfahrensvariante a) setzt man beispielsweise das $N^{\alpha A1}$, $N^{\alpha B1}$-Bis-Boc-Derivat eines Des-Octapeptid-(B23-30)-Insulin analog der im US—Patent 4 029 642 beschreibenen Verfahrensweise direkt mit einem Äquivalent einer Verbindung der Formel III um, wobei als Kondensationsmittel Dicyclohexylcarbodiimid im geringen Unterschuß in Anwesenheit von 1-Hydroxybenzotriazol dient.

Da bei dieser Verfahrensvariante gewöhnlich kein Schutz der Carboxylgruppe erfoderlich ist, entfällt normalerweise auch die Schädigung des Insulin-Derivates sowohl bei der Veresterung als auch bei der

alkalischen Versiefung. Nicht umgesetztes Des-Octapeptid und ein Peptid, das durch Kondensation von IV an $Asp^{A21}$-OH entsteht, sind aufgrund der unterschiedlichen Molekülargröße und Ladungszahl leicht durch Verteilungschromatographie an Sephadex®-LH 20 oder durch Gelchromatographie an Sephadex®-G 75 oder G 50 superfine abtrennbar.

Zur Abspaltung der tert.-Butylschutzgruppen muß das Reaktionsprodukt nur mit Trifluoressigsäure während 30—60 Minuten bei Raumtemperatur behandelt werden. Diese Reaktion schädigt das Insulin-Derivat nicht. Wählt man als N-Schutzgruppe den Methylsulfonylethyl-oxy-carbonylrest, so ist zur Abspaltung durch β-Eliminierung eine Alkalibehandlung notwendig. Die Reaktionsbedingungen sind so (z.B. 0,1 N NaOH, 0°C, 5 sek.), daß das Insulin-Derivat nicht geschädigt wird. Das als Ausgangsprodukt verwendete $N^{\alpha A1}$, $N^{\alpha B1}$-Bis-Boc-Des-$B_{23-30}$-Octa-peptid-Insulin vom Schwein stellt man beispielsweise auf folgendem Wege her:

Schweineinsulin wird in einer Mischung aus Dimethylformamid, Dimethylsulfoxid und Wasser in Anwesenheit von N-Ethylmorpholin mit überschüssigem tert.-Butyloxycarbonyl-N-hydroxy-succinimidester umgesetzt. Dabei entsteht das zu erwartende $N^{\alpha A1}$, $N^{\alpha B1}$, $N^{\varepsilon B29}$-Tris-Boc-Insulin.

Man gibt nun zu der Lösung dieser Verbindung in Dimethylformamid und Tris-Puffer (pH 7,5) Trypsin in kleinen Portionen so lange zu, bis in der Elektrophorese kein Ausgangsprodukt mehr zu erkennen ist. Das $N^{\alpha A1}$, $N^{\alpha B1}$-Bis-Boc-Des-$B_{23-30}$-Octapeptid-Insulin wird durch Verteilungschromatographie an Sephadex® LH 20 gereinigt.

Diese Verbindung wird nun mit einem Mol des Peptids der Formel III, das in an sich bekannter Weise nach den Methoden der Peptidchemie hergestellt wird, 1—2 Mol 1-Hydroxybenzotriazol und etwa 0,9 Mol Dicyclohexylcarbodiimid in Dimethylformamid bei etwa pH 7—8 zur Reaktion gebracht (vgl. Chem. Ber. *103* (1970), Seite 788).

Ds Rohprodukt wird durch Verteilungschromatographie gereinigt und durch Behandeln mit Trifluoressigsäure/Anisol bei Raumtemperatur von den Schutzgruppen befreit. nach Fällung mit Ether, isoelektrischer Fällung aus Wasser und Chromatographie an Sephadex®-G 75 oder G 50 superfine ist die Verbindung elektrophoretisch rein und kann in bekannter Wiese zur Kristallisation gebracht werden. Das so gewonnene Insulin-Derivat ist biologisch voll aktiv.

Des-$Phe^{B1}$-Insuline als Ausgangsverbindungen sind beispielsweise aus der DE—B—20 05 658 oder aus der EP—A—46 979 bekannt.

Die bei der Verfahrensvariante b) als Ausgangsverbindungen verwendeten Des-B30-Insuline sind beispielsweise aus der EP—A—46 979 oder Hoppe-Seyler's Z. Physiol. Chem. *359* (1978) 799 bekannt. Das bei Variante b) verwendete Ausgangsmaterial der Formel IV wird in an sich bekannter Weise nach den Methoden der Peptidchemie hergestellt.

Das Des-B30-Insulin und die Verbindung der Formel IV werden in Analogie zu der im US—Patent 4 320 196 beschreibenen Verfahrensweise in Gegenwart von Trypsin oder einer trypsinähnlichen Endopeptidase in einem organisch-wäßrigen Lösmittel-System bei pH 5—9 und einer Temperatur von 20 bis 40°C miteinander kondensiert. Das erhaltene Insulin-Derivat kann nach den gebräuchlichen Methoden der Peptidchemie isoliert werden.

Weiterhin können die Insulin-Derivate der Formel I analog zu den beispielsweise in der GB—A—2 069 502, EP—A—56 951, WO 82/4069, DE—A—31 29 404 und WO 83/1074 beschriebenen semisynthetischen Verfahrensweisen zur Herstellung der bekannten Insulin-(B30)-ester synthetisiert werden.

Aminosäureester der Formel IV, in denen $R^{31}$ für eine Alkylpolyoxyethylenoxykette steht, werden in Analogie zu der in der EP—A—27 161 beschreibenen Verfahrensweise hergestellt. Andere Verbindungen der Formel IV sind bekannt oder können in Analogie zu bekannten Verfahren hergestellt werden.

Alle die genannten Insulin-Derivate der Formel I haben gemeinsam, daß durch die Blockierung der (B30)-Carboxygruppe das Molekül effektiv eine zusätzliche positive Ladung erhält, die ihm einen in Richtung Neutralpunkt verschobenen isoelektrischen Punkt verleihen. Je nach Derivat werden isoelektrische Punkte zwischen 5,8 und 7,3 in der isoelektrischen Fokussierung gemessen. Damit sind die Derivate im Neutralbereich weniger löslich als natives Insulin oder Proinsulin, die ihren isoelektrischen Punkt und damit den Bereich maximaler Unlöslichkeit bei pH = 5,4 haben, während sie im Neutralbereich normalerweise gelöst vorliegen.

Die Löslichkeitseigenschaften von Insulin und Proinsulin lassen sich im Bereich oberhalb des isoelektrischen Punktes, d.h. im therapeutisch besonders interesseanten Neutralbereich, durch Zugabe von Zinkionen beeinflussen. Zink wirkt dabei als Depotprinzip in der Weise, daß es den hexameren Zustand des Insulins sowie dessen Kristallisierungstendenz stabilisiert. Im subkutanen Gewebe lösen sich diese Aggregate wieder.

Ein weiteres geläufiges Depoprinzip ist die Kristallisation des Insulins oder Proinsulins als Komplex mit einem basischen Protein, z.B. Globin oder Protamin.

Bei der Anwendung des Proinsulins in Lösung oder in Verbindung mit einem der beschreibenen Depotprinzipien bedarf es eines weiteren proteolytischen Abbaus, um natives, voll wirksames Insulin freizusetzen. Intaktes Proinsulin hat nur etwa 1/8 der biologischen Wirksamkeit des Insulins, weil, so die Vorstellung, ein Teil der biologisch aktiven Oberflächenregion, die Rezeptor-Bindungsregion, durch das im Proinsulin vorhandene C-Peptid maskiert ist. Als Proinsulin kommt allerdings für die Diabetestherapie nur homologes, also nur solches mit menschlicher Sequenz, in Frage (vgl. z.B. DE—A1—3232 036). Heterologes Proinsulin besitzt eine signifikante Immunogenität. Es ist in diesem Zusammenhang bemerkenswert, daß

auch humane Proinsuline Variationen im C-Peptidteil aufweisen können.

Überraschenderweise wurde nun gefunden, daß die Insulin-Derivate der Formel I, deren B-Kette C-terminal blockiert ist, im Unterschied zum Proinsulin in äquimolarer Menge eine etwa gleich hohe biologische Aktivität wie natives Insulin aufweisen.

Die erfindungsgemäßen Arzneimittel stellen vollkommen neue Verzögerungsprinzipien dar, die ohne Depot-Hilfstoffe, wie Zink oder Protaminsulfat, zur Wirkung gebracht werden können. Die Depotwirkung geht auf ein inhärentes, proteinchemisch bedingtes, physikalisches Prinzip, die Schwerlöslichkeit des Insulin-Derivats an dessen isoelektrischem Punkt, zurück. Seine Wiederauflösung unter physiologischen Bedingungen wird möglicherweise durch Abspaltung der zusätzlichen Gruppe(n) erreicht, z.B. durch Enzyme mit Esterase-Aktivität. Die jeweils abspaltbare(n) Gruppe(n) sind entweder rein physiologische Metaboliten oder aber leicht metabolisierbare, physiologisch unbedenkliche Substanzen.

Die Insulin-Derivate als Wirkstoffe dieser neuen Arzneimittel sind auch im Unterschied zu in der Literatur beschriebenen Intermediaten, die noch Teile des heterologen C-Peptids enthalten, in der Regel nicht stärker immunogen als das entsprechende Insulin selbst.

Die erfindungsgemäßen Mittel enthalten als Wirkstoff eines oder mehrere der Insulin-Derivate der Formel I.

Sie weisen vorzugsweise einen pH-Wert zwischen 2,5 und 8,5 auf, enthalten ein geeignetes Isotoniemittel, ein geeignetes Konservierungsmittel und gegebenenfalls einen geeigneten Puffer für einen pH-Bereich zwischen 5,0 und 8,5.

Eine typische Anwendungsform der beschriebenen Derivate stellen Präparationen dar, die unterhalb des isoelektrischen Punktes als Lösungen in einem physiologisch unbedenklichen Träger vorliegen. Dabei kann der pH der Lösung typischerweise pH = 4,5 betragen, liegt also deutlich höher als der von sauren Altinsulinen (typischerweise pH = 3,0). Eine neutralere Injektionslösung bietet u.U. deutliche Vorteile in Bezug auf deren Verträglichkeit.

Eine weitere typische Anwendungsform sind Suspensionen aus amorphen oder kristallinen Niederschlägen der beschreibenen Derivate in einem physiologisch unbedenklichen Träger von etwa neutralem pH.

Es ist aber auch möglich, die in den Derivaten inhärente Schwerlöslichkeit im physiologischen pH-Bereich durch zusätzliche Depotprinzipien, wie etwa durch Zugabe von Zink oder Protaminsulfat, zu verstärken. Die zugesetzte Zinkmenge kann dabei bis zu 100 µg $Zn^{2+}$/100 Insulineinheiten, typischerweise etwa 50 µ $Zn^{2+}$/100 Insulineinheiten, betragen. Die Protaminmenge kann zwischen 0,28 mg und 0,6 mg pro 100 Einheiten betragen (bezogen auf Protaminsulfat). Auf diese Weise sind besonders lang wirksame Präparationen herstellbar, für die es in Zukunft breitere Anwendung als bishcer geben wird, nachdem gerade eine Basalmenge an Insulin therapeutisch vorteilhaft erscheint. Dies ist schon jetzt eine Erkenntnis aus der Therapie mit Insulindosiergeräten.

Als physiologisch unbedenkliches und mit dem Insulinderivat verträgliches Trägermedium eignet sich eine sterile wäßrige Lösung, die zu Blut in der üblichen Weise, z.B. durch Glycerin, Kochsalz, Glukose, isotonisch gemacht wird und daneben noch eines der gebräuchlichen Konservierungsmittel z.B. Phenol, m-Kresol oder p-Hydroxybenzoesäureester, enthält. Das Trägermedium kann zusätzlich eine Puffersubstanz, z.b. Natriumacetate, Natriumcitrat, Natriumphosphat, enthalten. Zur Einstellung des pH werden verdünnte Säuren (typischerweise HCl) bzw. Laugen (typischerweise NaOH) verwendet.

Die Insulin-Derivate können in den erfindungsgemäßen Mitteln auch als Alkali- oder als Ammoniumsalze eingesetzt werden. Ein beliebiger Anteil eines oder mehrerer Insulin-Derivate der Formel I oder ein Insulin-Derivat der Formel I kann in einer Mischung weitere dieser Insulin-Derivate unabhängig voneinander jeweils in gelöster, amorpher und/oder kristalliner Form vorliegen.

Es ist manchmal vorteilhaft, der erfindungsgemäßen Zubereitung eine geeignete Menge eines geeigneten Stabilisators zuzusetzen, der die Präzapitation von Protein bei thermischmechanischer Belastung bei Kontakt mit verschiedenen Materialien verhindert. Solche Stabilisatoren sind beispielsweise aus der EP—A—18 609, der DE—A—32 40 177 oder aus der WO—A—83/00288 bekannt.

Bei den erfindungsgemäßen Mitteln, die auch eines der bekannten Verzögerungsprinzipien, wie etwa Protaminsulfat, Globin oder Zink in geeigneten Mengen enthalten können, kann ein solches Verzögerungsprinzip in Kombination mit den gesamten Wirkstoffanteil oder mit Teilen davon oder einem oder mehreren Insulin-Derivaten der Formel I in einer Mischung angewandt werden. Ein Mittel kann verschiedene Insulin-Derivate der Formel I in Kombination mit mehreren verschiedenen verzögernd wirkenden Hilfsstoffen enthalten.

Die erfindungsgemäßen Arzneimittel können neben den Insulin-Derivaten der Formel I auch natives Insulin und/oder Proinsulin und/oder Des-Phe-Insulin unabhängig voneinander jeweils in gelöster, amorpher und/oder kristalliner Form enthalten.

Mit den erfindungsgemäßen therapeutischen Mitteln sind also offenkundig vielfältige und sehr fein abgestimmte Wirkungscharakteristiken erzielbar; damit sollten nach den eingangs gemachten Bemerkungen Forstschritte insbesondere im Hinblick auf diabetische Spätkomplikationen verbunden sein.

Zur weiteren Erläuterung der Erfindung sollen die folgenden Beispiele dienen:

Herstellungsbeispiel 1

Humaninsulin-(B30)—O—$CH_2$—$CH_2$—$CH_3$

5 g Schweineinsulin werden in 45 ml Dimethylformamid, 25 ml Dimethylsulfoxid, 0,5 ml N-Ethylmorpholin und 2,5 ml Wasser gelöst. Unter Rühren gibt man bei Raumtemperatur 1,5 g tert.-Butyloxycarbonyl-N-hydroxysuccinimid zu und läßt 6 Stunden reagieren. Dann wird durch Zugabe eines Tropfens Eisessig gestoppt, das Produkt mit Ether gefällt und abfiltriert. Der Rückstand wird in 360 ml Dimethylformamid gelöst und mit 320 ml Tris-Puffer (0,05 M, 0,01 M an $CaCl_2$, pH 7,5) verdünnt. Bei 36°C werden im Abstand von je 1 Stunde Portionen von 20 mg Trypsin zugegeben.

Nach insgesamt 12 Zugaben stellt man mit Essigsäure auf pH 4,5 und dampft die Lösung ein. Die anschließende Reinigung des Materials an einer Sephadex®-LH 20-Säule (8 × 200 cm) mittels Verteilungschromatographie im System n-Butanol-Eisessig-Wasser (2:1:10) ergibt 3,25 g $N^{\alpha A1}$, $N^{\alpha B1}$-Bis-Boc-$Des_{23-30}$-Octapeptid-Insulin (Schwein), das in der sauren und basischen Elektrophorese kein Ausangsmaterial mehr zeigt. Die Aminosäureanalyse der Substanz ist korrekt. Nach einer probeweisen Abspaltung der Boc-Gruppen ist keine Insulinaktivität mehr zu finden. Dieses Material (3,25 g) wird in 30 ml Dimethylformamid zusammen mit 100 mg 1-Hydroxybenzotriazol, 750 mg HCl·Gly-Phe-Phe-Tyr($Bu^t$)-Thr-Pro-Lys(Boc)-Thr($Bu^t$)-$OCH_2$—$CH_2$—$CH_3$ und 0,5 ml N-Ethylmorpholin gelöst. Dann gibt man bei Raumtemperatur 120 mg Dicyclohexylcarbodiimid zu und rührt die Reaktion 24 Stunden. Der abgeschiedene Dicyclohexylharnstoff wird abfiltriert, das Produkt durch Etherzugabe gefällt.

Den Niederschlag filtriert man ab, wäscht mit Ether und trocknet. Die Substanz wird durch Verteilungschromatographie an Sephadex®-LH 20 im obigen System vorgereinigt. 2,6 g Material aus dem Hauptpeak isoliert man durch Fällung mit Aceton/Ether. Das getrocknete, noch ungeschützte Derivat läßt man mit einem Gemisch aus 5 ml Trifluoressigsäure und 1 ml Anisol 60 Minuten bei Raumtemperatur reagieren. Aus der mit eis gekühlten Lösung fällt man anschließend die Rohsubstanz durch Zugabe von Ether. Den getrockneten Niederschlag löst man in Wasser, präzipitiert mit wäßrigem Ammoniak und zentrifugiert. Die Reinigung des Produktes erfolgt in 10%iger Essigsäure über Sephadex®-G 50 superfine oder G 75. Aus den Fraktionen des gewünschten Peaks kann man das Humaninsulin-(B30)-$OCH_2CH_2CH_3$ durch Gefriertrocknung isolieren (Ausbeute nach Kristallisation: 1,2 g). Das so gewonnene Insulin-Derivat zeigt im biologischen Test eine dem Humaninsulin äquivalente Wirksamkeit.

Die Herstellung des Octapeptids der Formel III erfolgt gemäß folgendem Kondensationsschema nach üblichen-Peptid-Kondensationsmethoden:

Synthgeseschema für das Octapeptid der Formel III

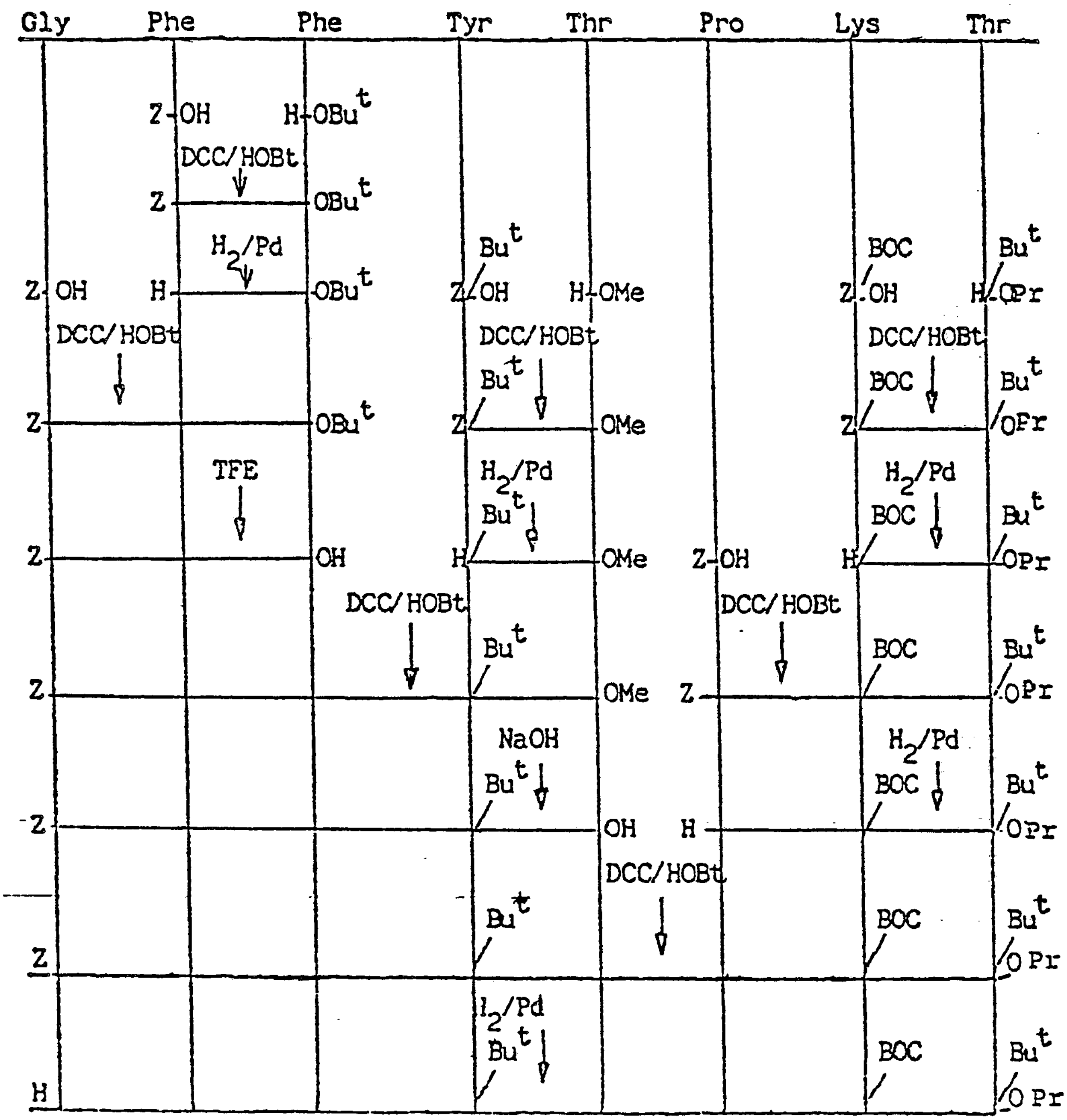


Aminosäure- und Elementaranalyse entsprechen der Theoric

Arzneimittel

Beispiel 1

Insulin-(B30)-$OCH_3$ vom Schwein (Semisynthetisch aus Des-B30-Schweineinsulin hergestellt) in schwach saurer, gelöster Formulierung mit 40 I.E. pro ml und deren Depot-Wirksamkeit:

Man löst in einem Gesamtvolumen von 10 ml Wasser

| | |
|---|---|
| Insulin-(B30)-$OCH_3$ vom Schwein (27,5 I.E./mg) | 14,5 mg |
| Traubenzucker (Monohydrat) krist. | 540,0 mg |
| p-Hydroxybenzoesäuremethylester | 10,0 mg |

Der pH-Wert wird durch Zugabe von 1N HCl bzw. 1N NaOH auf pH = 4,5 eingestellt.

Eine solche Lösung zeigt bei einer Dosierung von 0,4 I.E./kg am Kaninchen eine ausgeprägte Depot-Wirksamkeit. Die Fläche unter der Blutzuckerkurve ist gleich wie die eines Standard-präparates mit 40 I.E./ml.

Beispiel 2

Humaninsulin-$Thr^{B30}(Bu^t)OBu^t$ (Pr = 6,8), hergestellt durch Semisynthese aus Schweineinsulin, in neutraler Formulierung mit 40 I.E. pro ml und deren Depot-Wirksamkeit:

Man löst in einem Gesamtvolumen von 10 ml Wasser

| | |
|---|---|
| Humaninsulin-$Thr^{B30}(Bu^t)OBu^t$ (27 I.E./mg) | 14,8 mg |
| Natriumdihydrogenphosphat-dihydrat | 21,0 mg |
| m-Kresol | 27,0 mg |
| Glycerin | 160,0 mg |

Der pH-Wert wird durch Zugabe von 1N HCl bzw. 1N NaOH auf pH = 7,3 eingestellt.

Eine solche Suspension zeigt bei einer Dosierung von 0,4 I.E./kg am Kaninchen eine ausgeprägte Depot-Wirksamkeit.

Beispiel 3

Humaninsulin-(B30)-$NH_2$ hergestellt aus Schweineinsulin durch Semisynthese, in Form einer kristallinen NPH-Zubereitung mit 40 I.E./ml und deren stark verzögerte Wirkung:

Man löst in einem Gesamt-Volumen von 10 ml mit Wasser:

| | |
|---|---|
| Humaninsulin-(B30)-$NH_2$ (27 I.E./mg) | 14,5 mg |
| Protaminsulfat | 1,3 mg |
| Natriumdihydrogenphosphat-dihydrat | 21,0 mg |
| m-Kresol | 15,0 mg |
| Phenol | 6,0 mg |
| Glycerin | 160,0 mg |

Der pH-Wert wird durch Zugabe von 1N HCl bzw. 1N NaOH auf pH = 7,3 eingestellt.

Eine solche Kristallsuspension zeigt bei einer Dosierung von 0,4 I.E./kg am Kaninchen eine stark verzögerte Wirkung.

Beispiel 4

Michung von Humaninsulin-$Arg^{B31}$-OH und Humaninsulin-$Thr^{B30}Bu^t(OBu^t)$ beide hergestellt durch Semisynthese aus Schweineinsulin, in Form einer zinkhaltigen Suspension mit 40 I.E./ml und deren stark verzögerte Wirkung:

Man löst in einem Gesamt-Volumen von 10 ml mit Wasser:

| | |
|---|---|
| Humaninsulin-$Arg^{B31}$-OH (27,5 I.E./mg) | 7,3 mg |
| Humaninsulin-$Thr^{B30}Bu^t(OBu^t)$ (27,0 I.E./mg) | 7,4 mg |
| Zinkchlorid, wasserfrei | 0,46 mg |
| Natriumacetat | 14,0 mg |
| p-Hydroxybenzoesäuremethylester | 10,0 mg |
| Kochsalz | 80,0 mg |

Der pH-Wert wird durch Zugabe von 1N HCl bzw. 1N NaOH auf pH = 7,0 eingestellt.

Eine solche Suspension zeigt bei einer Dosierung von 0,4 I.E./kg am Kaninchen eine stark verzögerte Wirkung.

Beispiel 5

Humaninsulin-(B30)—O—$(CH_2CH_2—O—)_{20}C_2H_5$, hergestellt durch Semisynthese aus Schweininsulin, in Form einer neutralen Zubereitung mit 100 I.E./ml und deren verzögerte Wirkung:

Man löst in einem Gesamt-Volumen von 10 ml mit Wasser:

| | |
|---|---|
| Humaninsulin-(B30)—O—$(CH_2CH_2—O—)_{20}C_2H_5$ (24,0 I.E./mg) | 41,7 mg |
| Natriumacetat | 14,0 mg |
| p-Hydroxybenzoesäuremethylester | 10,0 mg |
| Kochsalz | 80,0 mg |

Durch Zugabe von 1N HCl bzw. 1N NaOH wird pH = 7,0 eingestellt.
Eine solche Suspension zeigt am Kaninchen eine verzögerte Wirkung.

**Patentansprüche für die Vertragsstaaten: BE CH DE FR GB IT LI LU NL SE**

1. Arzneimittel mit verzögerter Wirkung zur Behandlung von Diabetes mellitus, bestehend aus mindestens einem Insulin-Derivat der Formel I

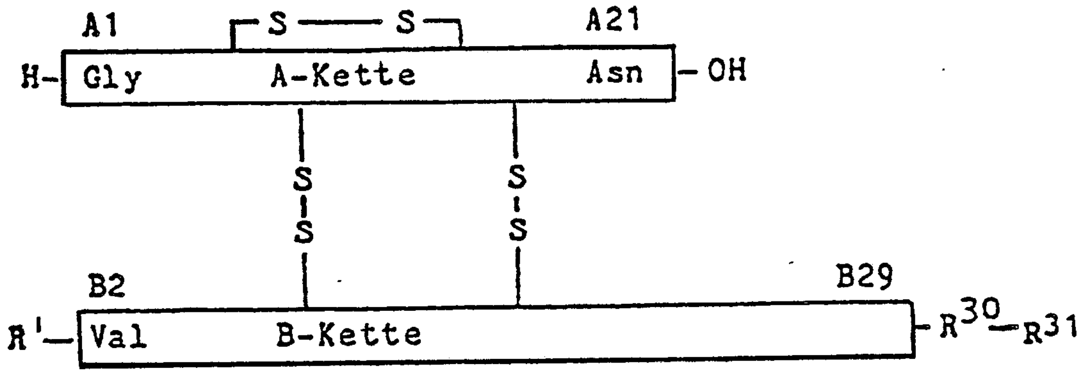

(I)

in welcher

$R^1$ H oder H-Phe bedeutet,

$R^{30}$ für den Rest einer neutralen, genetisch kodierbaren L-Aminosäure, deren gegebenenfalls vorhandene OH-Gruppe frei oder durch eine physiologisch verträgliche Gruppe geschützt vorliegen kann, steht und

$R^{31}$ eine physiologisch verträgliche, die Carboxygruppe blockierende neutrale Gruppe bedeutet, wobei jedoch Des-B30-Humaninsulin-$Leu^{B30}$-$NH_2$, Des-B30-Humaninsulin-$Gly^{B30}$-$NH_2$ und Des-B30-Humaninsulin-$Phe^{B30}$-$NH_2$ ausgenommen sind

und einem pharmazeutisch unbedenklichen Träger.

2. Mittel nach Anspruch 1, dadurch gekennzeichnet, daß in dem Insulin-Derivat der Formel I $R^1$ = H-Phe ist.

3. Mittel nach einem der Ansprüche 1 oder 2, dadurch gekennzeichnet, daß in dem Insulin-Derivat der Formel I $R^{30}$ für Ala, Thr oder Ser steht.

4. Mittel nach einem oder mehreren der Ansprüche 1 bis 3, dadurch gekennzeichnet, daß in dem Insulin-Derivat der Formel I die A-Kette und die Kette (B2-29) die Sequenz des Humaninsulins aufweisen.

5. Mittel nach einem oder mehreren der Ansprüche 1 bis 4, dadurch gekennzeichnet, daß in dem Insulin-Derivat der Formel I $R^{31}$ eine Gruppe der Formel $—NR^aR^b$ oder $—OR^c$, bedeutet, worin $R^a$ und $R^b$ gleich oder verschieden sind und für Wasserstoff, $(C_1—C_6)$-Alkyl, $(C_3—C_8)$-Cycloalkyl, $(C_6—C_{10})$-Aryl, $(C_7—C_{11})$-Aralkyl, $(C_3—C_9)$-Heteroaryl oder $—(CH_2—CH_2—O)_mR$ mit m = 1 bis etwa 120 und R = $(C_1—C_4)$-Alkyl stehen, wobei dieser Gruppen im Alkylteil auch durch einen oder mehrere gleiche oder verschiedene Substituenten aus der Reihe Halogen, Nitro, $(C_1—C_4)$-Alkoxy, Methylendioxy und $(C_1—C_4)$-Alkyl substituiert sein können oder $R^a$ und $R^b$ gemeinsam für $—[CH_2]_n$ mit n = 4—6 stehen, worin eine Methylengruppe auch durch O oder NH ersetzt sein kann, und $R^c$ für $(C_1—C_6)$-Alkyl, $(C_3—C_8)$-Cycloalkyl, $(C_6—C_{10})$-Aryl, $(C_7—C_{11})$-Aralkyl, $(C_3—C_9)$-Heteroaryl oder den vorstehend definierten Rest $(CH_2CH_2—O—)_mR$ steht, wobei die Arylreste wie bei $R^a$ und $R^b$ substituiert sein können.

6. Mittel nach einem odere mehreren der Ansprüche 1 bis 5, dadurch gekennzeichnet, daß in dem Insulin-Derivat der Formel I $R^{31}$ für $—O—(CH_2—CH_2—O—)_mR$ steht, wobei m und R wie im Anspruch 5 definiert sind.

7. Mittel nach einem oder mehreren der Ansprüche 1 bis 6, dadurch gekennzeichnet, daß in dem Insulin-Derivat der Formel I $R^{31}$ für $(C_1—C_6)$-Alkoxy oder $(C_7—C_{11})$-Aralkoxy steht.

8. Mittel nach einem oder mehreren der Ansprüche 1 bis 7, dadurch gekennzeichnet, daß in dem Insulin-Derivat der Formel I eine in $R^{30}$ gegebenenfalls vorhanden OH-Gruppe frei oder durch $(C_1—C_6)$-Alkyl, $(C_1—C_6)$-Alkanoyl oder $(C_7—C_{11})$-Aralkyl geschützt vorliegt.

9. Mittel gemäß einem oder mehreren der Ansprüche 1 bis 8, dadurch gekennzeichnet, daß dieses einen pH-Wert zwischen 2,5 und 8,5 aufweist, ein geeignetes Isotonie-Mittel und ein geeignetes Konservierungsmittel enthält und daß das Insulin-Derivat der Formel I gelöst und/oder in Suspension vorliegt.

10. Mittel gemäß einem oder mehreren der Ansprüche 1 bis 9, dadurch gekennzeichnet, daß dieses einen geeigneten Puffer enthält und der pH-Wert zwischen 5,0 und 8,5 liegt.

11. Mittel gemäß einem oder mehreren der Ansprüche 1 bis 10, dadurch gekennzeichnet, daß dieses zwischen 0 und 100 µg Zink/100 I.U. enthält.

12. Mittel gemäß einem oder mehreren der Ansprüche 1 bis 11, dadurch gekennzeichnet, daß der Wirkstoff der Formel I in Form eines Alkalisalzes oder des Ammoniumsalzes vorliegt.

13. Mittel gemäß einem oder mehreren der Ansprüche 1 bis 12, dadurch gekennzeichnet, daß ein beliebiger Anteil eines oder mehrere Insulin-Derivat der Formel I in einer Mischung weiterer dieser Insulin-Derivate unabhängig voneinander jeweils in gelöster, amorpher und/oder kristalliner Form vorliegen.

14. Mittel gemäß einem oder mehreren der Ansprüche 1 bis 13, dadurch gekennzeichnet, daß dieses eine geeignete Menge eines Hilfsmittels mit verzögernder Wirkung enthält.

15. Mittel gemäß Anspruch 14, dadurch gekennzeichnet, daß dieses Verzögerungsprinzip in Kombination mit dem gesamten Wirkstoffanteil oder mit Teilen davon oder einem oder mehreren Insulin-Derivaten der Formel I in einer Mischung angewandt wird.

16. Mittel gemäß einem oder mehreren der Ansprüche 1 bis 15, dadurch gekennzeichnet, daß es verschiedene Insulin-Derivate der Formel I in Kombination mit mehreren verschiedenen verzögernd wirkenden Hilfsstoffen enthält.

17. Verwendung oder Insulin-Derivate der Formel I gemäß der Definition in einem oder mehreren der Ansprüche 1 bis 8 zur Herstellung eines Arzneimittels zur Behandlung des Diabetes mellitus.

**Patentansprüche für den Vertragsstaat: AT**

1. Verfahren zur Herstellung eines Arzneimittels mit verzögerter Wirkung zur Behandlung von Diabetes mellitus, dadurch gekennzeichnet, daß man mindestens ein Insulin-Derivat der Formel I

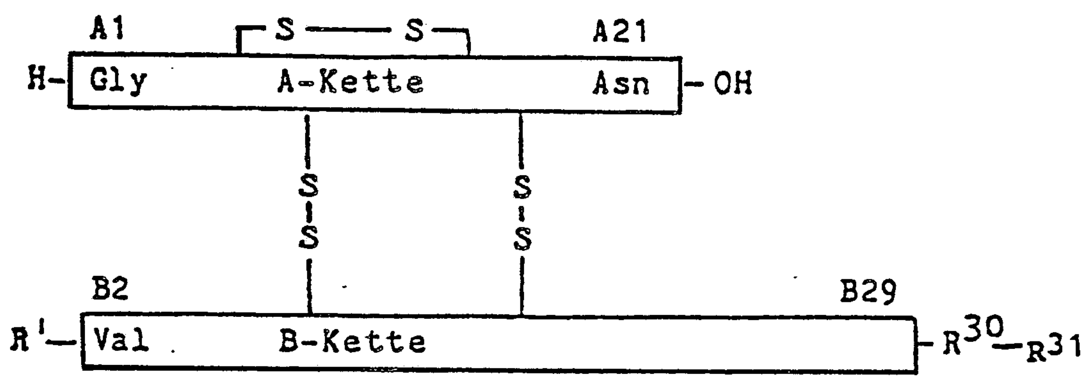

(I)

in welcher

$R^1$ H oder H-Phe bedeutet,

$R^{30}$ für den Rest einer neutralen, genetisch kodierbaren L-Aminosäure, deren gegebenenfalls vorhandene OH-Gruppe frei oder durch eine physiologisch verträgliche Gruppe geschützt vorliegen kann, steht und

$R^{31}$ eine physiologisch verträgliche, die Carboxygruppe blockierende neutrale Gruppe bedeutet, wobei jedoch Des-B30-Humaninsulin-$Leu^{B30}$-$NH_2$, Des-B30-Humaninsulin-$Gly^{B30}$-$NH_2$ und Des-B30-Humaninsulin-$Phe^{B30}$-$NH_2$ ausgenommen sind

mit einem pharmazeutisch unbedenklichen Träger in eine geeignete Darreichungsform bringt.

2. Verfahren nach Anspruch 1, dadurch gekennzeichnet, daß man ein Insulin-Derivat der Formel I mit $R^1$ = H-Phe einsetzt.

3. Verfahren nach einem der Ansprüche 1 oder 2, dadurch gekennzeichnet, daß man ein Insulin-Derivat der Formel I mit $R^{30}$ = Ala, Thr oder Ser einsetzt.

4. Verfahren nach einem oder mehreren der Ansprüche 1 bis 3, dadurch gekennzeichnet, daß man ein Insulin-Derivat der Formel I einsetzt, in welchem A-Kette und die Kette (B2-29) die Sequenz des Humaninsulins aufweisen.

5. Verfahren nach einem oder mehreren der Ansprüche 1 bis 4, dadurch gekennzeichnet, daß man ein Insulin-Derivat der Formel I mit $R^{31}$ = Gruppe der Formel $—NR^aR^b$ oder $—OR^c$ einsetzt, worin $R^a$ und $R^b$ gleich oder verschieden sind und für Wasserstoff, ($C_1$—$C_6$)-Alkyl, ($C_3$—$C_8$)-Cycloalkyl, ($C_6$—$C_{10}$)-Aryl, ($C_7$—$C_{11}$)-Aralkyl, ($C_3$—$C_9$)-Heteroaryl oder $—(CH_2—CH_2—O)_mR$ mit m = 1 bis etwa 120 und R = ($C_1$—$C_4$)-Alkyl stehen, wobei dieser Gruppen im Alkylteil auch durch einen oder mehrere gleiche oder verschiedene Substituenten aus der Reihe Halogen, Nitro, ($C_1$—$C_4$)-Alkoxy, Methylendioxy und ($C_1$—$C_4$)-Alkyl substituiert sein können oder $R^a$ und $R^b$ gemeinsam für $—[CH_2]_n—$ mit n = 4—6 stehen, worin eine Methylengruppe auch durch O oder NH ersetzt sein kann, und $R^c$ für ($C_1$—$C_6$)-Alkyl, ($C_3$—$C_8$)-Cycloalkyl, ($C_6$—$C_{10}$)-Aryl, ($C_7$—$C_{11}$)-Aralkyl, ($C_3$—$C_9$)-Heteroaryl oder den vorstehend definierten Rest $(CH_2CH_2—O—)_mR$ steht, wobei die Arylreste wie bei $R^a$ und $R^b$ substituiert sein können.

6. Verfahren nach einem oder mehreren der Ansprüche 1 bis 5, dadurch gekennzeichnet, daß man ein Insulin-Derivat der Formel I mit $R^{31}$ = $—O—(CH_2—CH_2—O—)_mR$ einsetzt, wobei m und R wie im Anspruch 5 definiert sind.

7. Verfahren nach einem oder mehreren der Ansprüche 1 bis 6, dadurch gekennzeichnet, daß man ein Insulin-Derivat der Formel I mit $R^{31}$ = ($C_1$—$C_6$)-Alkoxy oder ($C_7$—$C_{11}$)-Aralkoxy einsetzt.

8. Verfahren nach einem oder mehreren der Ansprüche 1 bis 7, dadurch gekennzeichnet, daß man ein Insulin-Derivat der Formel I einsetzt, worin eine in $R^{30}$ gegebenenfalls vorhanden OH-Gruppe frei oder durch ($C_1$—$C_6$)-Alkyl, ($C_1$—$C_6$)-Alkanoyl oder ($C_7$—$C_{11}$)-Aralkyl geschützt vorliegt.

9. Verfahren gemäß einem oder mehreren der Ansprüche 1 bis 8, dadurch gekennzeichnet, daß man

ein Arzneimittel herstellt, welches einen pH-Wert zwischen 2,5 und 8,5 aufweist, ein geeignetes Isotonie-Mittel und ein geeignetes Konservierungsmittel und das Insulin-Derivat der Formel I gelöst und/oder in Suspension enthält.

10. Verfahren gemäß einem oder mehreren der Ansprüche 1 bis 9, dadurch gekennzeichnet, daß man ein Arzneimittel herstellt, welches einen geeigneten Puffer enthält und einen pH-Wert zwischen 5,0 und 8,5 aufweist.

11. Verfahren gemäß einem oder mehreren der Ansprüche 1 bis 10, dadurch gekennzeichnet, daß man ein Arzneimittel herstellt, welches zwischen 0 und 100 µg Zink/100 I.U. enthält.

12. Verfahren gemäß einem oder mehreren der Ansprüche 1 bis 11, dadurch gekennzeichnet, daß man ein Arzneimittel mit dem Wirkstoff der formel I in Form eines Alkalisalzes oder des Ammoniumsalzes herstellt.

13. Verfahren gemäß einem oder mehreren der Ansprüche 1 bis 12, dadurch gekennzeichnet, daß man ein Arzneimittel mit einem beliebigen Anteil eines oder mehrerer Insulin-Derivate der Formel I in einer Mischung weiterer dieser Insulin-Derivate unabhängig voneinander jeweils in gelöster, amorpher und/oder kristalliner Form herstellt.

14. Verfahren gemäß einem oder mehreren der Ansprüche 1 bis 13, dadurch gekennzeichnet, daß man ein Arzneimittel herstellt, welches noch eine geeignete Menge eines Hilfsmittels mit verzögernder Wirkung enthält.

15. Verfahren gemäß Anspruch 14, dadurch gekennzeichnet, daß dieses Verzögerungsprinzip in Kombination mit dem gesamten Wirkstoffanteil oder mit Teilen davon oder einem oder mehreren Insulin-Derivaten der Formel I in einer Mischung angewandt wird.

16. Verfahren gemäß einem oder mehreren der Ansprüche 1 bis 15, dadurch gekennzeichnet, daß man ein Arzneimittel mit verschiedenen Insulin-Derivaten der Formel I in Kombination mit mehreren verschiedenen verzögernd wirkenden Hilfsstoffen herstellt.

17. Verwendung der Insulin-Derivate der Formel I gemäß der Definition in einem oder mehreren der Ansprüche 1 bis 8 zur Herstellung eines Arzneimittels zur Behandlung des Diabetes mellitus.

**Revendications pour les Etats contractants: BE CH DE FR GB IT LI LU NL SE**

1. Médicament à effet retard, pour le traitement du diabète sucré, constitué d'au moins un dérivé d'insuline de formule I

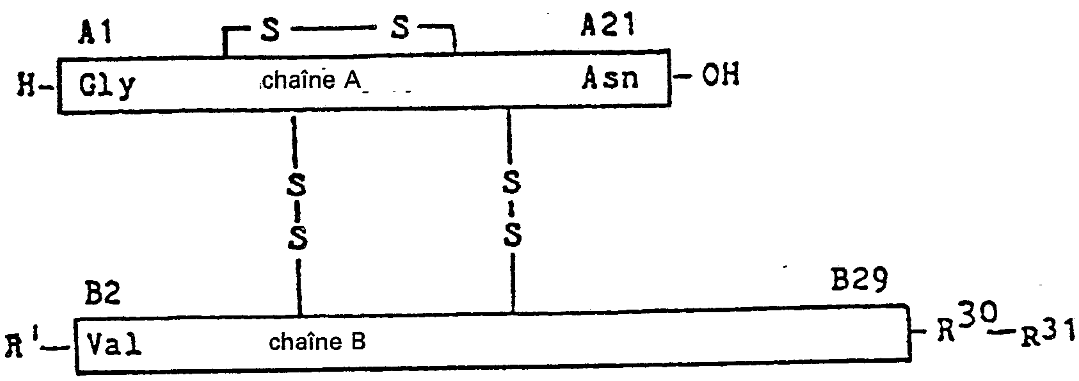


(I)

dans laquelle

$R^1$ représente H ou H-Phe,

$R^{30}$ représente le reste d'un L-aminoacide neutre, génétiquement codable, dont un groupe OH éventuellement présent peut se trouver sous forme libre ou être protégé par un groupe physiologiquement acceptable, et

$R^{31}$ représente un groupe neutre physiologiquement acceptable bloquant le groupe carboxy,

à l'exception toutefois de la des-B30-insuline humaine-$Leu^{B30}$-$NH_2$, de la des-B30-insuline humaine-$Gly^{B30}$-$NH_2$ et de la des-B30-insuline humaine-$Phe^{B30}$-$NH_2$,

et d'un véhicule pharmaceutiquement acceptable.

2. Médicament selon la revendication 1, caractérisé en ce que, dans le dérive d'insuline de formule I, $R^1$ = H-Phe.

3. Médicament selon l'une des revendications 1 ou 2, caractérisé en ce que, dans le dérivé d'insuline de formule I, $R^{30}$ représente Ala, Thr ou Ser.

4. Médicament selon une ou plusieurs des revendications 1 à 3, caractérisé en ce que, dans le dérivé d'insuline de formule I, la chaîne A et la chaîne (B 2—29) comportent la séquence de l'insuline humaine.

5. Médicament selon une ou plusieurs des revendications 1 à 4, caractérisé en ce que, dans le dérivé d'insuline de formule I, $R^{31}$ représente un groupe de formule $—NR^aR^b$ ou $—OR^c$, dans lesquelles $R^a$ et $R^b$ sont identiques ou différents et représentent un atome d'hydrogène ou un groupe alkyle en $C_1—C_6$, cycloalkyle en $C_3—C_8$, aryle en $C_6—C_{10}$, aralkyle en $C_7—C_{11}$, hétéroaryle en $C_3—C_9$ ou $—(CH_2—CH_2—O—)_mR$ avec m = 1 à environ 120 et R = alkyle en $C_1—C_4$, ces groupes pouvant encore être substitués sur le

fragment alkyle par un ou plusieurs substituants identiques ou différents, choisis parmi des atomes d'halogène et des groupes nitro, alcoxy en $C_1$—$C_4$, méthylènedioxy et alkyle en $C_1$—$C_4$, ou $R^a$ et $R^b$ représentent ensemble $[CH_2]_n$ avec n = 4—6, un groupe méthylène pouvant également être remplacé par O ou NH, et $R^c$ représente un groupe alkyle en $C_1$—$C_6$, cycloalkyle en $C_3$—$C_8$, aryle en $C_6$—$C_{10}$, aralkyle en $C_7$—$C_{11}$, hétéroaryle en $C_3$—$C_9$ ou le radical $(CH_2CH_2—O—)_mR$ défini précédemment, les restes aryle pouvant être substitués comme indiqué pour $R^a$ et $R^b$.

6. Médicament selon une ou plusieurs des revendications 1 à 5, caractérisé en ce que, dans le dérivé d'insuline de formule I, $R^{31}$ représente $—O—(CH_2—CH_2—O—)_mR$, m et R étant tels que définis dans la revendication 5.

7. Médicament selon une ou plusieurs des revendications 1 à 6, caractérisé en ce que, dans le dérivé d'insuline de formule I, $R^{31}$ représente un groupe alcoxy en $C_1$—$C_6$ ou aralcoxy en $C_7$—$C_{11}$.

8. Médicament selon une ou plusieurs des revendications 1 à 7, caractérisé en ce que, dans le dérivé d'insuline de formule I, un groupe OH éventuellement présent dans $R^{30}$ est sous forme libre ou est protégé par un groupe alkyle en $C_1$—$C_6$, alcanoyle en $C_1$—$C_6$ ou aralkyle en $C_7$—$C_{11}$.

9. Médicament selon une ou plusieurs des revendications 1 à 8, caractérisé en ce que celui-ci présente un pH compris entre 2,5 et 8,5, contient un agent d'isotonic approprié et un conservateur approprié, et en ce que le dérivé d'insuline de formule I se trouve en solution et/ou en suspension.

10. Médicament selon une ou plusieurs des revendications 1 à 3, caractérisé en ce que celui-ci contient un tampon approprié et le pH est compris entre 5,0 et 8,5.

11. Médicament selon une ou plusieurs des revendications 1 à 10, caractérisé en ce que celui-ci contient entre 0 et 100 µg de zinc/100 U.I.

12. Médicament selon une ou plusieurs des revendications 1 à 11, caractérisé en ce que la substance active de formule I se trouve sous forme d'un sel alcalin ou de sel d'ammonium.

13. Médicament selon une ou plusieurs des revendications 1 à 12, caractérisé en ce qu'une proportion quelconque d'un ou plusieurs dérivés d'insuline de formule I se trouve dans un mélange d'autres de ces dérivés d'insuline, chacun, indépendamment les uns des autres, sous forme dissoute, amorphe et/ou cristalline.

14. Médicament selon une ou plusieurs des revendications 1 à 13, caractérisé en ce que celui-ci contient une quantité appropriée d'un agent auxiliaire à effet retard.

15. Médicament selon la revendication 14, caractérisé en ce que ce principe retard est utilisé en association avec la quantité totale de substances actives ou avec des parties de celles-ci ou avec un ou plusieurs dérivés d'insuline de formule I en un mélange.

16. Médicament selon une ou plusieurs des revendications 1 à 15, caractérisé en ce qu'il contient divers dérivés d'insuline de formule I en association avec plusieurs substances auxiliaires différentes à effet retard.

17. Utilisation des dérivés d'insuline de formule I selon la définition dans une ou plusieurs des revendications 1 à 8, pour la fabrication d'un médicament pour le traitement du diabète sucré.

**Revendications pour l'Etat contractant: AT**

1. Procédé pour la préparation d'un médicament à effet retard pour le traitement du diabète sucré, caractérisé en ce que l'on met sous une forme pharmaceutique appropriée au moins un dérivé d'insuline de formule I

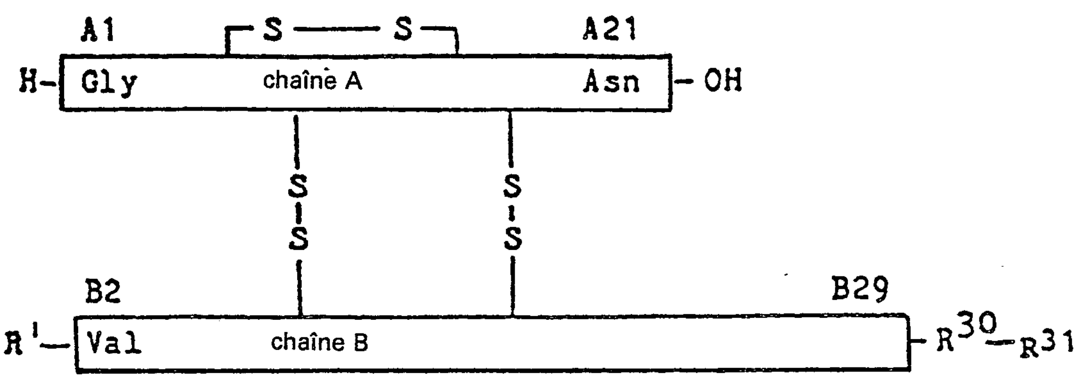

(I)

dans laquelle

$R^1$ représente H ou H-Phe,

$R^{30}$ représente le reste d'un L-aminoacide neutre, génétiquement codable, dont un groupe OH éventuellement présent peut se trouver sous forme libre ou être protégé par un groupe physiologiquement acceptable, et

$R^{31}$ représente un groupe neutre physiologiquement acceptable bloquant le groupe carboxy, à l'exception toutefois de la des-B30-insuline humaine-$Leu^{B30}$-$NH_2$, de la des-B30-insuline humaine-$Gly^{B30}$-$NH_2$ et de la des-B30-insuline humaine-$Phe^{B30}$-$NH_2$,

avec un véhicule pharmaceutiquement acceptable.

2. Procédé selon la revendication 1, caractérisé en ce que, l'on utilise un dérivé d'insuline de formule I dans lequel $R^1$ = H-Phe.

3. Procédé selon l'une des revendications 1 ou 2, caractérisé en ce que l'on utilise un dérivé d'insuline de formule I dans lequel $R^{30}$ = Ala, Thr ou Ser.

4. Procédé selon une ou plusieurs des revendications 1 à 3, caractérisé en ce que, l'on utilise un dérivé d'insuline de formule I dans lequel la chaîne A et la chaîne (B 2—29) comportent la séquence de l'insuline humaine.

5. Procédé selon une ou plusieurs des revendications 1 à 4, caractérisé en ce que l'on utilise dérivé d'insuline de formule I, $R^{31}$ représente un groupe de formule $—NR^aR^b$ ou $—OR^c$, dans lesquelles $R^a$ et $R^b$ sont identiques ou différents et représentent un atome d'hydrogène ou un groupe alkyle en $C_1—C_6$, cycloalkyle en $C_3—C_8$, aryle en $C_6—C_{10}$, aralkyle en $C_7—C_{11}$, hétéroaryle en $C_3—C_9$ ou $—(CH_2—CH_2—O—)_mR$ avec m = 1 à environ 120 et R = alkyle en $C_1—C_4$, ces groupes pouvant encore être substitués sur le fragment alkyle par un ou plusieurs substituants identiques ou différents, choisis parmi des atomes d'halogène et des groupes nitro, alcoxy en $C_1—C_4$, méthylènedioxy et alkyle en $C_1—C_4$, ou $R^a$ et $R^b$ représentent ensemble $[CH_2]_n$ avec n = 4—6, un groupe méthylène pouvant également être remplacé par O ou NH, et $R^c$ représente un groupe alkyle en $C_1—C_6$, cycloalkyle en $C_3—C_8$, aryle en $C_6—C_{10}$, aralkyle en $C_7—C_{11}$, hétéroaryle en $C_3—C_9$ ou le radical $(CH_2CH_2—O—)_mR$ défini précédemment, les restes aryle pouvant être substitués comme indiqué pour $R^a$ et $R^b$.

6. Procédé selon une ou plusieurs des revendications 1 à 5, caractérisé en ce que l'on utilise un dérivé d'insuline de formule I dans lequel $R^{31}$ représente $—O—(CH_2—CH_2—O—)_mR$, m et R étant tels que définis dans la revendication 5.

7. Procédé selon une ou plusieurs des revendications 1 à 6, caractérisé en ce que l'on utilise un dérivé d'insuline de formule I dans lequel $R^{31}$ représente un groupe alcoxy en $C_1—C_6$ ou aralcoxy en $C_7—C_{11}$.

8. Procédé selon une ou plusieurs des revendications 1 à 7, caractérisé en ce que l'on utilise un dérivé d'insuline de formule I dans lequel un groupe OH éventuellement présent dans $R^{30}$ est sous forme libre ou est protégé par un groupe alkyle en $C_1—C_6$, alcanoyle en $C_1—C_6$ ou aralkyle en $C_7—C_{11}$.

9. Procédé selon une ou plusieurs des revendications 1 à 8, caractérisé en ce que l'on fabrique un médicament qui présente un pH compris entre 2,5 et 8,5, contient un agent d'isotonie approprié et un conservateur approprié et le dérivé d'insuline de formule I en solution et/ou en suspension.

10. Procédé selon une ou plusieurs des revendications 1 à 9, caractérisé en ce que l'on fabrique un médicament qui contient un tampon approprié et présente un pH compris entre 5,0 et 8,5.

11. Procédé selon une ou plusieurs des revendications 1 à 10, caractérisé en ce que l'une fabrique un médicament qui contient entre 0 et 100 µg de zinc/100 U.I.

12. Procédé selon une ou plusieurs des revendications 1 à 11, caractérisé en ce que l'on fabrique un médicament avec la substance active de formule I sous forme d'un sel alcalin ou du sel d'ammonium.

13. Procédé selon une ou plusieurs des revendications 1 à 12, caractérisé en ce que l'on fabrique un médicament avec une proportion quelconque d'un ou plusieurs dérivés d'insuline de formule I dans un mélange d'autres de ces dérivés d'insuline, chacun, indépendamment les uns des autres, sous forme dissoute, amorphe et/ou cristalline.

14. Procédé selon une ou plusieurs des revendications 1 à 13, caractérisé en ce que l'on fabrique un médicament qui contient en outre une quantité appropriée d'un agent auxiliaire à effet retard.

15. Procédé selon la revendication 14, caractérisé en ce que ce l'on utilise ce principe retard en association avec la quantité totale de substances actives ou avec des parties de celles-ci ou avec un ou plusieurs dérivés d'insuline de formule I en un mélange.

16. Procédé selon une ou plusieurs des revendications 1 à 15, caractérisé en ce que l'on fabrique un médicament avec divers dérivés d'insuline de formule I en association avec plusieurs substances auxiliaires différentes à effet retard.

17. Utilisation des dérivés d'insuline de formule I selon la définition dans une plusieurs des revendications 1 à 8, pour la fabrication d'un médicament pour le traitement du diabète sucré.

**Claims for the Contracting States: BE CH DE FR GB IT LI LU NL SE**

1. A medicament having a delayed action for the treatment of diabetes mellitus, comprising at least one insulin derivative of the formula I

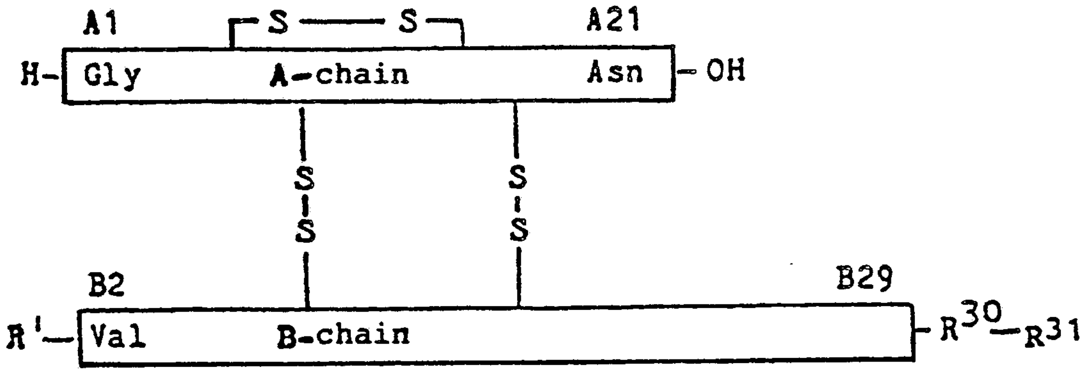

(I)

in which

$R^1$ denotes H or H-Phe

$R^{30}$ represents the radical of a neutral, genetically codable L-aminoacid whose OH group, where present, can be free or protected by a physiologically acceptable group, and

$R^{31}$ denotes a physiologically acceptable neutral group blocking the carboxyl group, with the exception of des-B30 human insulin $Leu^{B30}$-$NH_2$ and des-B30 human insulin $Gly^{B30}$-$NH_2$ and des-B30 human insulin $Phe^{B30}$-$NH_2$ and a pharmaceutically acceptable carrier.

2. An agent as claimed in claim 1, wherein $R^1$ represents H-Phe in the insulin derivative of the formula I.

3. An agent as claimed in either of claims 1 or 2 wherein $R^{30}$ represents Ala, Thr or Ser in the insulin derivative of the formula I.

4. An agent as claimed in one or more of claims 1 to 3, wherein, in the insulin derivative of the formula I, the A chain and the chain (B2—29) have the sequence of human insulin.

5. The process as claimed in one or more of claims 1 to 4, which comprises using an insulin derivative of the formula I, in which $R^{31}$ denotes a group of the formula $—NR^aR^b$ or $—OR^c$, wherein

$R^a$ and $R^b$ are identical or different and represent hydrogen, ($C_1$—$C_6$)-alkyl, ($C_3$—$C_8$)-cycloalkyl, ($C_6$—$C_{10}$)-aryl, ($C_7$—$C_{11}$)-aralkyl, ($C_3$—$C_9$)-heteroaryl or $—(CH_2—CH_2—O)_mR$ in which m = 1 to about 120 and R = ($C_1$—$C_4$)-alkyl, and these groups may furthermore be substituted in the alkyl part by one or more identical or different substituents from the series comprising halogen, nitro, ($C_1$—$C_4$)-alkoxy, methylenedioxy and ($C_1$—$C_4$)-alkyl or $R^a$ and $R^b$ together denote $[CH_2]_n$ where n = 4—6 and a methylene group may also be replaced by O or NH, and $R^c$ represents ($C_1$—$C_6$)-alkyl, ($C_3$—$C_8$)-cycloalkyl, ($C_6$—$C_{10}$)-aryl, ($C_7$—$C_{11}$)-aralkyl, ($C_3$—$C_9$)-heteroaryl or the above-defined radical $(CH_2CH_2—O—)_mR$, wherein, as in the case of $R^a$ and $R^b$, the aryl radicals may be substituted.

6. An agent as claimed in one or more of claims 1 to 5, wherein in the insulin derivative of the formula I, $R^{31}$ represents $—O—(CH_2—CH_2—O—)_mR$, wherein m and R are as defined in claim 5.

7. An agent as claimed in one or more of claims 1 to 6, wherein, in the insulin derivative of the formula I, $R^{31}$ represents ($C_1$—$C_6$)-alkoxy or ($C_7$—$C_{11}$)-aralkoxy.

8. An agent as claimed in one or more of claims 1 to 7, wherein, in the insulin derivative of the formula I, any OH group which may be present in $R^{30}$ is free or protected by ($C_1$—$C_6$)-alkyl, ($C_1$—$C_6$)-alkanoyl or ($C_7$—$C_{11}$)-aralkyl.

9. An agent as claimed in one or more of claims 1 to 8, wherein the insulin derivative of the formula I is present in dissolved form and/or in suspension.

10. An agent as claimed in one or more of claims 1 to 9, which contains a suitable buffer and has a pH between 5.0 and 8.5.

11. An agent as claimed in one or more of claims 1 to 10, which contains between 0 and 100 µg of zinc/100 I.U.

12. An agent as claimed in one or more of claims 1 to 11, wherein the active compound of the formula I is present in the form of an alkali metal salt or of the ammonium salt.

13. An agent as claimed in one or more of claims 1 to 12, wherein any desired amount of one or more insulin derivatives of the formula I is present in a mixture of other insulin derivatives from amongst these, independently of one another and in each case in dissolved, amorphous and/or crystalline form.

14. An agent as claimed in one or more of claims 1 to 13, which contains a suitable amount of an auxiliary having a delaying action.

15. An agent as claimed in claim 14, wherein this delayed-action principle is used in combination with the total amount of active compound or with some of this, or with one or more insulin derivatives of the formula I, in a mixture.

16. An agent as claimed in one or more of claims 1 to 15, which contains different insulin derivatives of the formula I in combination with several different auxiliaries having a delaying action.

17. Use of the insulin derivatives of the formula I defined in one or more of claims 1 to 8, for the preparation of a medicament for the treatment of diabetes mellitus.

**Claims for the Contracting State: AT**

1. A process for the preparation of a medicament having a delayed action for the treatment of diabetes mellitus, which comprises bringing into a suitable dosage form at least one insulin derivative of the formula I

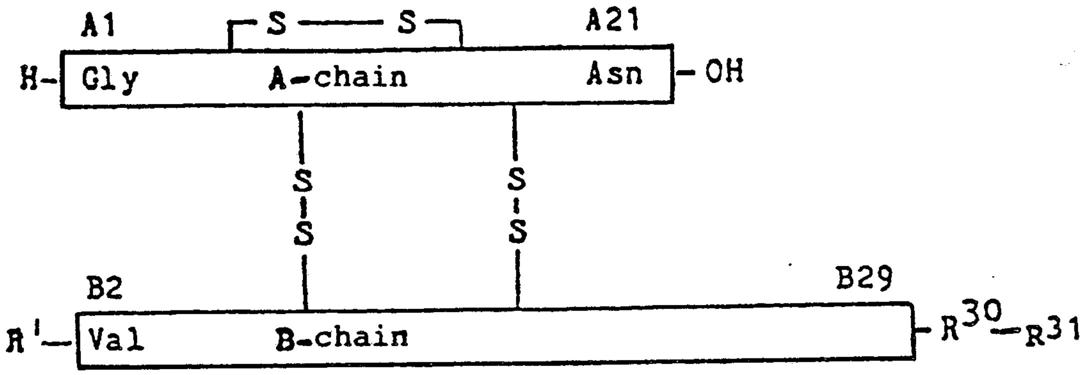

(I)

in which

$R^1$ denotes H or H-Phe

$R^{30}$ represents the radical of a neutral, genetically codable L-aminoacid whose OH group, where present, can be free or protected by a physiologically acceptable group, and

$R^{31}$ denotes a physiologically acceptable neutral group blocking the carboxyl group, with the exception of des-B30 human insulin $Leu^{B30}$-$NH_2$ and des-B30 human insulin $Gly^{B30}$-NH, and des-B30 human insulin $Phe^{B30}$-$NH_2$ $Phe^{B30}$-$NH_2$ and a pharmaceutically acceptable carrier.

2. The process as claimed in claim 1, which comprises using an insulin derivative of the formula I in which $R_1$ = H-Phe.

3. The process as claimed in either of claims 1 or 2, which comprises using an insulin derivative of the formula I in which $R^{30}$ = Ala, Thr or Ser.

4. The process as claimed in one or more of claims 1 to 3, which comprises using an insulin derivative of the formula I in which the A chain and the chain (B2—29) have the sequence of human insulin.

5. The process as claimed in one or more of claims 1 to 4, which comprises using an insulin derivative of the formula I, in which $R^{31}$ denotes a group of the formula $—NR^aR^b$ or $—OR^c$, wherein

$R^a$ and $R^b$ are identical or different and represent hydrogen, $(C_1—C_6)$-alkyl, $(C_3—C_8)$-cycloalkyl, $(C_6—C_{10})$-aryl, $(C_7—C_{11})$-aralkyl, $(C_3—C_9)$-heteroaryl or $—(CH_2—CH_2—O)_mR$ in which m = 1 to about 120 and R = $(C_1—C_4)$-alkyl, and these groups may furthermore be substituted in the alkyl part by one or more identical or different substituents from the series comprising halogen, nitro, $(C_1—C_4)$-alkoxy, methylenedioxy and $(C_1—C_4)$-alkyl or $R^a$ and $R^b$ together denote $[CH_2]_n$ where n = 4—6 and a methylene group may also be replaced by O or NH, and $R^c$ represents $(C_1—C_6)$-alkyl, $(C_3—C_8)$-cycloalkyl, $(C_6—C_{10})$-aryl, $(C_7—C_{11})$-aralkyl, $(C_3—C_9)$-heteroaryl or the above-defined radical $(CH_2CH_2—O—)_mR$, wherein, as in the case of $R^a$ and $R^b$, the aryl radicals may be substituted.

6. The process as claimed in one or more of claims 1 to 5 which comprises using an insulin derivative of the formula I in which $R^{31}$ denotes $—O—(CH_2—CH_2—O—)_mR$, wherein m and R are as defined in claim 5.

7. The process as claimed in one or more of claims 1 to 6, which comprises using an insulin derivative of the formula I in which $R^{31}$ denotes $(C_1—C_6)$-alkoxy or $(C_7—C_{11})$-aralkoxy.

8. The process as claimed in one or more of claims 1 to 7, which comprises using an insulin derivative of the formula I in which any OH group which may be present in $R^{30}$ is free or protected by $(C_1—C_6)$-alkyl, $(C_1—C_6)$-alkanoyl or $(C_7—C_{11})$-aralkyl.

9. The process as claimed in one or more of claims 1 to 8, which comprises preparing a medicament which has agent and a suitable preservative and the insulin derivative of the formula I in dissolved form and/or in suspension.

10. The process as claimed in one or more of claims 1 to 9, which comprises preparing a medicament which contains a suitable buffer and has a pH between 5.0 and 8.5.

11. The process as claimed in one or more of claims 1 to 10, which comprises preparing a medicament which contains between 0 and 100 µg of zinc/100 I.U.

12. The process as claimed in one or more of claims 1 to 11, which comprises preparing a medicament having the active compound of the formula I in the form of an alkali metal salt or of the ammonium salt.

13. The process as claimed in one or more of claims 1 to 12, which comprises preparing a medicament having any desired amount of one or more insulin derivatives of the formula I in a mixture of other insulin derivatives from amongst these, independently of one another and in each case in dissolved, amorphous and/or crystalline form.

14. The process as claimed in one or more of claims 1 to 13, which comprises preparing a medicament which contains a suitable amount of an auxiliary having a delaying action.

15. The process as claimed in claim 14, which comprises using this delayed-action principle in combination with the total amount of active compound or with some of this, or with one or more insulin derivatives of the formula I, in a mixture.

16. The process as claimed in one or more of claims 1 to 15, which comprises preparing a medicament having different insulin derivatives of the formula I in combination with several different auxiliaries having a delaying action.

17. Use of the insulin derivatives of the formula I defined in one or more of claims 1 to 8, for the preparation of a medicament for the treatment of diabetes mellitus.